# EUROPÄISCHE PATENTSCHRIFT

(11) **EP 0 515 934 B1**
(45) Veröffentlichungstag und Bekanntmachung des Hinweises auf die Patenterteilung: **09.09.1998**
(21) Anmeldenummer: 92108270.7
(22) Anmeldetag: 15.05.1992
(51) Int. Cl.: C07D 231/22, A61K 31/415, C07D 231/56, C07D 231/28

(54) **Verwendung von 1-Thiocarbamoyl 5-hydroxy-pyrazolen als Mikrobizide, 1-Thiocarbamoyl 5-hydroxy-pyrazole und Verfahren zu ihrer Herstellung**
Microbial application of 1-thiocarbamoyl-5-hydroxy-pyrazoles, 1-thiocarbamoyl-5-hydroxy pyrazoles and process for their fabrication
Application microbicide de thiocarbamoyl-1-hydroxy-5-pyrazoles, thiocarbamoyl-1-hydroxy-5-pyrazoles et procédé pour leur fabrication

(30) Priorität: 28.05.1991 DE 4117385
(43) Veröffentlichungstag der Anmeldung: 02.12.1992
(73) Patentinhaber: BAYER AG, 51368 Leverkusen (DE)
(72) Erfinder: Sasse, Klaus, Dr., W-5060 Bergisch Gladbach 2 (DE); Wachtler, Peter, Dr., W-5000 Köln 80 (DE); Ludwig, Georg-Wilhelm, Dr., D-4150 Krefeld 1 (DE); Paulus, Wilfried, Dr., D-4150 Krefeld 1 (DE)

(56) Entgegenhaltungen:
- PATENT ABSTRACTS OF JAPAN vol. 3, no. 138 (C-64)16. November 1979
- PATENT ABSTRACTS OF JAPAN vol. 3, no. 138 (C-64)16. November 1979
- ARCHIV DER PHARMAZIE Bd. 316, Nr. 1, 1. Januar 1983, WEINHEIM Seiten 2 - 6; KURT WEGNER: 'Synthese und H2-antihistaminische Wirksamkeit von 2-Amidino-1,1-dihydro-3H-pyrazol-3-onen'
- SCIENTIA PHARMACEUTICA Bd. 51, Nr. 2, 30. Juni 1983, WIEN Seiten 167 - 172; K. WEGNER: 'N-[2-((5-Methyl-4-Imidazolyl)methylt hio)ethyl-2,5-dihydro-5-oxo-1H- pyrazol-1-carboxa midine-Synthese und H2-antihistaminische Wirksamkeit'

## Beschreibung

Die Erfindung betrifft die Verwendung von 1-Thiocarbamoyl-5-hydroxy-pyrazolen der Formel 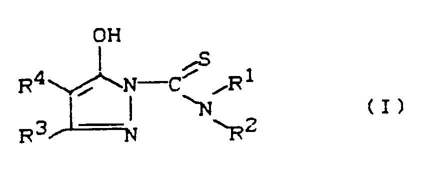 worin die Substituenten die im Hauptanspruch angegebene Bedeutung besitzen, als Mikrobizide zum Schutz technischer Materialien, neue 4-substituierte 1-Thiocarbamoyl-5-hydroxy-pyrazole und ein Verfahren zur ihrer Herstellung durch Kondensation von α-Formylsäureestern oder -amiden oder von β-Ketoessigsäureestern oder -amiden mit Thiosemicarbaziden.

Aus der US-PS 4 663 327 sind 1-Hetaryl-4-aryl-pyrazolin-5-one, z.B. 1-[Pyri(mi)dyl-(2)]-4-phenyl-pyrazolin-5-one und deren mikrobizide Eigenschaften bekannt. Da die Verbindungen jedoch farbig sind und bei ihrer Einarbeitung in technische Materialien, z.B. Anstrichmittel und Kunststoffe, zu Verfärbungen führen, sind sie trotz ihrer guten mikrobiziden Eigenschaften für den Materialschutz nicht verwendbar.

Bislang werden zur fungiziden Ausrüstung von Kunststoffen noch arsenorganische Verbindungen eingesetzt, obgleich der Ersatz dieser Verbindungen und auch der noch in Anstrichmitteln verwendeten quecksilberorganischen Verbindungen aus ökotoxikologischen Gründen sehr erwünscht ist. Bislang wurden jedoch noch keine ökotoxikologisch günstigeren Verbindungen gefunden, die die hohen Anforderungen erfüllen, die an für die mikrobizide Ausrüstung von Kunststoffen verwendbare Mikrobizide gestellt werden müssen. Solche Mikrobizide müssen nämlich zusätzlich zu einer guten mikrobiziden Wirksamkeit auch noch eine hohe Temperaturbeständigkeit aufweisen und dürfen außerdem die Eigenschaften der Kunststoffe nicht beeinträchtigen, z.B. diese nicht verfärben. Diese letzte Anforderung gilt auch für Mikrobizide, die in Anstrichmitteln eingesetzt werden sollen.

Überraschenderweise wurde gefunden, daß 1-Thiocarbamoyl-5-hydroxy-pyrazole die Nachteile des Standes der Technik nicht aufweisen und alle an solche Wirkstoffe gestellten Anforderungen erfüllen. Obwohl sie ökotoxikologisch unbedenklich sind, erreichen sie die Wirkung der unerwünschten arsen- und quecksilberorganischen Verbindungen. Sie besitzen die nötige thermische Stabilität und beeinträchtigen die Eigenschaften, wie Farbe und Elastizität, der mit ihnen ausgerüsteten Kunststoffe, Anstrichmittel und Anstriche nicht negativ.

Gegenstand der Erfindung ist also die Verwendung von Verbindungen der Formel I, worin
- R¹ und R²: unabhängig voneinander Wasserstoff oder gegebenenfalls substituiertes Alkyl,
- R³: Wasserstoff oder gegebenenfalls substituiertes Alkyl, Alkenyl, Alkinyl, Cycloalkyl, Alkoxycarbonyl, Aralkyl oder Aryl,
- R⁴: Wasserstoff oder gegebenenfalls substituiertes Alkyl, Alkenyl, Alkinyl, Cycloalkyl, Alkoxy, Alkylthio, Aralkyl, Aralkoxy, Aralkylthio, Aryl, Aryloxy, Arylthio, Alkoxycarbonyl, Alkoxycarbonylmethyl oder Aminocarbonyl oder
- R³ und R⁴: zusammen einen 1,ω-C₃-C₆-Alk(en)ylenrest bedeuten,
als Mikrobizide zum Schutz technischer Materialien.

Bevorzugte Bedeutungen für die Substituenten R¹ bis R⁴ werden nachstehend erläutert:

Für Alkyl steht geradkettiges oder verzweigtes Alkyl mit vorzugsweise 1 bis 12 C-Atomen, wie Methyl, Ethyl, n- und i-Propyl, n-, sek.-, i- und tert.-Butyl, n-, i- und tert.-Pentyl, n-Hexyl, i-Octyl, i-Nonyl, n-Decyl und n- Dodecyl. Diese Alkylgruppen können durch 1 bis 3 Halogenatome, vorzugsweise Chlor und/oder Fluor, oder durch eine C₁-C₆-Alkoxycarbonylgruppe substituiert sein. Substituierte Alkylgruppen umfassen demnach beispielsweise Mono-, Di- und Trifluormethyl, Monochlordifluormethyl, Methoxy- und Ethoxycarbonylmethyl.

Unter den Begriff Alkoxy fällt geradkettiges und verzweigtes Alkoxy mit vorzugsweise 1 bis 12 C-Atomen, wie beispielsweise Methoxy, Ethoxy, n- und i-Propoxy, n-, i-, sek- und tert.-Butoxy, Hexoxy.

Alkenyl bedeutet geradkettiges und verzweigtes Alkenyl mit vorzugsweise 2 bis 6 C-Atomen, wie beispielsweise Vinyl und Allyl.

Alkinyl steht für geradkettiges oder verzweigtes Alkinyl mit vorzugsweise 2 bis 6 C-Atomen, wie beispielsweise Ethinyl, Propinyl und 3,3-Dimethylpropinyl; bevorzugte substituierte Alkinylgruppen sind insbesondere die Iod-substituierten Alkinyle wie z.B. 1-Iod-propinyl.

Cycloalkylgruppen umfassen Cycloalkyl mit vorzugsweise 5 bis 7 C-Atomen, wie beispielsweise Cyclohexyl; bevorzugte substituierte Cycloalkylgruppen umfassen durch 1 bis 3 C₁-C₄-Alkylgruppen oder 1 bis 3 Halogenatomen, wie Chlor und/oder Fluor, substituiertes Cycloalkyl, wie beispielsweise Methylcyclohexyl, Dimethylcyclohexyl, 1,3,3,-Trimethylcyclohexyl und 3-Chlorcyclohexyl.

Alkoxycarbonyl steht für geradkettiges oder verzweigtes Alkoxycarbonyl mit vorzugsweise 1 bis 6 C-Atomen im Alkoxyrest, wie beispielsweise Methoxycarbonyl, Ethoxycarbonyl, n- und i-Propoxycarbonyl, n-, i-, sek.- und tert.-Butoxycarbonyl, Hexoxycarbonyl. Analoges gilt für die Alkoxycarbonylmethylgruppen.

Aralkyl enthält vorzugsweise 1 bis 6, insbesondere 1 bis 4 C-Atome im geradkettigen oder verzweigten Alkylteil und vorzugsweise Phenyl oder Naphthyl als Arylteil. Beispiele für solche Aralkylgruppen umfassen Benzyl, α-Methylbenzyl, α,α-Dimethylbenzyl, 2-Phenethyl, α- und β-Naphthylmethyl. Diese Aralkylreste können 1 bis 3 Substituenten aus der Reihe Halogen (insbesondere Chlor und/oder Fluor), Nitro, Cyano, gegebenenfalls halogeniertes C₁-C₄-Alkyl oder -Alkoxy, wie beispielsweise Methyl, Ethyl, Trifluormethyl, Difluorchlormethyl, Difluormethyl, Trichlormethyl, Methoxy, Ethoxy, Trifluormethoxy, Difluorchlormethoxy und Difluormethoxy, gegebenenfalls halogeniertes C₁-C₄-Alkylmercapto, wie beispielsweise Methylmercapto, Trifluormethylmercapto, Difluorchlormethylmercapto tragen.

Unter dem Begriff Aryl ist unsubstituiertes oder substituiertes Aryl mit vorzugsweise 6 bis 10 C-Atomen im Arylteil zu verstehen. Bevorzugte Beispiele umfassen Phenyl und Naphthyl. Die Arylgruppen können 1 bis 3 Substituenten aus der Reihe Halogen (insbesondere Chlor und/oder Fluor), C₁-C₄-Alkyl oder -Alkoxy, Halogen-C₁-C₂-alkyl, (wie Trifluormethyl, Difluormethyl), Cyano, Nitro oder Amino tragen.

Unter dem Begriff Alkoxy ist geradkettiges und verzweigtes Alkoxy mit vorzugsweise 1 bis 6, insbesondere 1 bis 4 C-Atomen zu verstehen. Bevorzugte Beispiele umfassen Methoxy, Ethoxy, n- und i-Propoxy, n-, i-, sek.- und tert.-Butoxy sowie Hexoxy.

Alkylthio steht für geradkettiges oder verzweigtes Alkylthio mit vorzugsweise 1 bis 6 C-Atomen. Bevorzugte Beispiele umfassen Methylthio, Ethylthio, n- und i-Propylthio, n-, i-, sek.- und tert.-Butylthio, n-Pentylthio und seine Isomeren wie 1-, 2- und 3-Methyl-butylthio. Die Alkylthiogruppen können durch 1 bis 3 Halogenatome (vorzugsweise Chlor und/oder Fluor) substituiert sein; bevorzugte Beispiele hierfür sind Di- und Trifluormethylthio sowie Difluorchlormethylthio.

Aralkoxy enthält vorzugsweise 1 bis 6 C-Atome im geradkettigen oder verzweigten Alkylteil und vorzugsweise Phenyl als Arylteil. Bevorzugte Beispiele sind Benzyloxy und Phenethyloxy. Die Aralkoxygruppen können durch 1 bis 3 Halogenatome (vorzugsweise Chlor und/oder Fluor) oder durch eine C₁-C₄-Alkylgruppe substituiert sein.

Aralkylthio enthält vorzugsweise 1 bis 6 C-Atome im geradkettigen oder verzweigten Alkylteil und vorzugsweise Phenyl als Arylteil. Bevorzugtes Beispiel ist Benzylthio. Die Aralkylthiogruppen können durch 1 bis 3 Halogenatome (vorzugsweise Chlor und/oder Fluor) oder durch eine C₁-C₄-Alkylgruppe substituiert sein.

Aryloxy enthält vorzugsweise 1 bis 10 C-Atome im Arylteil. Bevorzugte Beispiele sind Phenoxy und Naphthoxy. Die Aryloxygruppen können 1 bis 3 Substituenten aus der Reihe Halogen (vorzugsweise Chlor und/oder Fluor), C₁-C₄-Alkyl, Halogen-C₁-C₂-alkyl (wie Di- und Trifluormethyl), Cyano, Nitro oder Amino tragen.

Arylthio enthält vorzugsweise 6 bis 10 C-Atome im Arylteil. Bevorzugte Beispiele sind Phenylthio und Naphthylthio. Die Arylthiogruppen können die unter "Aryloxy" aufgezählten Substituenten tragen.

Der Begriff Aminocarbonyl umfaßt beispielsweise unsubstituiertes Aminocarbonyl, N-Methylaminocarbonyl und N,N-Dimethylaminocarbonyl.

Bevorzugte Beispiele für 1,ω-C₃-C₆-Alk(en)ylenreste umfassen 1,3-Propylen, 1,4-Butylen und 1,4-Butadien(1,3)ylen.

Verbindungen der Formel (I), worin
- R¹: Wasserstoff oder gegebenenfalls substituiertes Alkyl, Aralkyl oder Aryl,
- R²: Wasserstoff,
- R³: Wasserstoff oder gegebenenfalls substituiertes Alkyl oder Aryl und
- R⁴: Wasserstoff oder gegebenenfalls substituiertes Alkyl, Cycloalkyl, Aralkyl, Alkoxycarbonyl oder Aryl bedeuten,
werden bevorzugt.

Verbindungen der Formel I, worin
- R¹-R³: Wasserstoff und
- R⁴: Wasserstoff oder gegebenenfalls substituiertes Alkyl, Aralkyl oder Aryl bedeuten,
werden besonders bevorzugt.

Die bevorzugtesten Verbindungen entsprechen der Formel I, wobei
- R¹-R³: Wasserstoff und
- R⁴: C₁-C₆-Alkyl, Benzyl oder Phenyl bedeuten.

Typische Vertreter der erfindungsgemäß zu verwendenden Verbindungen I sind in den Beispielen aufgeführt.

Die erfindungsgemäß zu verwendenden Verbindungen I können auch in ihrer tautomeren Pyrazol-5-on-Form vorliegen.

Die erfindungsgemäß zu verwendenden Verbindungen I und Verfahren zu ihrer Herstellung sind teilweise bekannt. So werden in den veröffentlichten japanischen Patentanmeldungen 79/115 374 und 79/119 031 3-mono- und 3,4-di-substituierte 1-Thiocarbamoyl-5-hydroxy-pyrazole beschrieben, die gegen Pflanzenkrankheiten wirksam sind. Insbesondere sollen sie fungizide Wirkung besitzen; vgl. auch J. Pesticide, Sci. 11, 205-212 (1986).

Aus Arch. Pharm. (Weinheim) 316, 2-6 (1983) und aus Sci. Pharm. 51 (2), 167-172 (1982) sind 4-mono- und 3,4-di-substituierte 1-Thiocarbamoyl-5-hydroxy-pyrazole mit antihistaminischer Wirkung bekannt.

Soweit die Verbindungen noch neu sind, können sie analog den bekannten Herstellungsverfahren hergestellt werden. Üblicherweise setzt man einen α-Formylsäureester oder ein α-Formylsäureamid oder ein β-Ketoessigsäureester oder ein β-Ketoessigsäureamid mit einem Thiosemicarbazid um. Diese Kondensationsreaktion verläuft nach folgendem Reaktionsschema (demonstriert am β-Ketoessigsäureethylester als β-Diketo-Ausgangsprodukt):

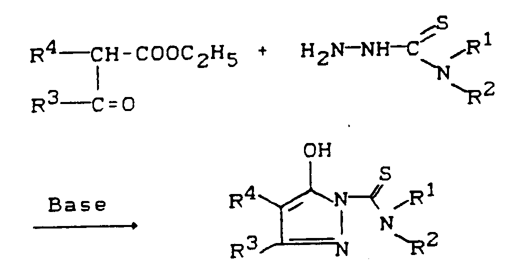

Die Substituenten R¹ bis R⁴ haben im obigen Reaktionsschema die oben zu den Verbindungen der Formel I angegebenen Bedeutungen.

Pro Mol α-Formylsäurederivat bzw. pro Mol β-Diketoverbindung setzt man vorzugsweise 0,8 bis 1,0 Mol Thiosemicarbazid zu.

Zur Erleichterung der Ringschlußreaktion setzt man vorteilhafterweise Basen wie Natriumhydroxid, Kaliumhydroxid oder Kalium-tert.-butylat zu. Vorzugsweise setzt man die Base in einer dem α-Formylsäurederivat bzw. der β-Diketoverbindung etwa äquivalenten Menge zu.

Die Kondensation kann gegebenenfalls in Gegenwart eines Lösungsmittels durchgeführt werden; als Lösungsmittel haben sich vor allem Alkohole wie Ethanol oder aromatische Kohlenwasserstoffe wie Toluol bewährt.

Die Kondensationsreaktion kann innerhalb eines größeren Temperaturbereichs ausgeführt werden. Für die zuerst ablaufende Thiosemicarbazonbildung kann bei Temperaturen von 20 bis 110°C gearbeitet werden, vorzugsweise zwischen 60 und 90°C. Die nach der Basenzugabe ablaufende Cyclokondensationsreaktion kann bei Temperaturen von 20 bis 100° , vorzugsweise 20 bis 40°C vorgenommen werden. Da die Basenzugabe in manchen Fällen exotherm verläuft, kann ein Kühlen bei diesem Reaktionsschritt erforderlich sein.

Die 1-Thiocarbamoyl-hydroxy-pyrazole können nach bekannten Methoden aus den Reaktionsgemischen isoliert werden. Man geht im allgemeinen so vor, daß man die Reaktionsgemische vom Lösungsmittel befreit und den Rückstand mit wäßriger Salzsäure behandelt. Die dabei ausfallenden Pyrazole werden durch Absaugen abgetrennt. Es ist jedoch auch möglich, das Reaktionsgemisch unmittelbar in einen großen Überschuß verdünnter Salzsäure einzugießen und die als Niederschlag sich abscheidenden Pyrazole abzufiltrieren.

Die für die Herstellung der erfindungsgemäß zu verwendenden Verbindungen I benötigten Ausgangsverbindungen, nämlich die α-Formylsäureester bzw. -amide und die gegebenenfalls in α-Stellung substituierten β-Ketoessigsäureester bzw. -amide und die gegebenenfalls substituierten Thiosemicarbazide, sind entweder bekannte Verbindungen oder in Analogie zu bekannten Verbindungen nach vorbeschriebenen Verfahren herstellbar.

Wie oben bereits gesagt, sind manche erfindungsgemäß zu verwendenden Verbindungen I neu. Weiterer Gegenstand der Erfindung sind daher Verbindungen der Formel 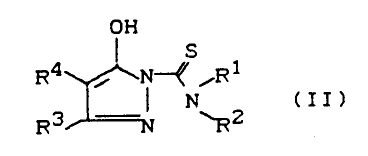 worin
- R¹ und R²: die im Hauptanspruch angegebene Bedeutung besitzen,
- R³: für Wasserstoff und
- R⁴: für gegebenenfalls substituiertes C₆-C₈-Alkyl, für gegebenenfalls substituiertes Aryl mit vorzugsweise 6 bis 10 C-Atomen, wobei Phenyl und 4-Chlorphenyl als Substituenten R⁴ ausgeschlossen sind, oder für gegebenenfalls substituiertes Aralkyl mit vorzugsweise 1 bis 6, insbesondere 1 bis 4 C-Atomen im geradkettigen oder verzweigten Alkylteil und 6 bis 10 C-Atomen im Arylteil mit Ausnahme von unsubstituiertem Benzyl stehen.

Die für R⁴ in Formel (II) verwendeten Begriffe "Alkyl", "Aryl", "Aralkyl" und deren gegebenenfalls vorhandenen Substituenten entsprechen im Rahmen der Beschränkungen der Formel (II) den oben für "Alkyl", "Aryl", "Aralkyl" und deren gegebenenfalls vorhandene Substituenten der Formel (I) angegebenen Bedeutungen.

Weiterer Gegenstand der Erfindung ist ein Verfahren zur Herstellung der Verbindung II, wonach man Verbindungen der Formeln 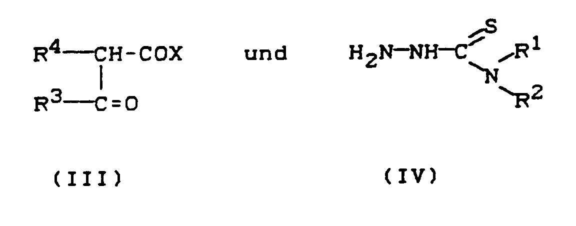 worin
- R¹ bis R⁴: die zu Formel II angegebene Bedeutung besitzen und
- X: für C₁-C₄-Alkoxy, Phenoxy oder Amino steht,
auf an sich bekannte Weise umsetzt.

Bei den Materialien, die durch die erfindungsgemäß zu verwendenden Verbindungen I geschützt werden können, handelt es sich um nicht lebende Materialien, die für die Verwendung in der Technik zubereitet worden sind. Beispielsweise können technische Materialien, die durch erfindungsgemäß zu verwendende Wirkstoffe vor mikrobieller Veränderung oder Zerstörung geschützt werden sollen, Klebstoffe, Leime, Papier und Karton, Textilien, Leder, Holz, Anstrichmittel, Kunststoffe und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, deren Funktion durch Vermehrung von Mikroorganismen beeinträchtigt werden kann. Im Rahmen der vorliegenden Erfindung werden unter technischen Materialien vorzugsweise Anstrichmittel, Kunststoffe, Kunststoffartikel und Holz verstanden.

Mikroorganismen, die einen Abbau oder eine Veränderung der technischen Materialien bewirken können, umfassen beispielsweise Bakterien, Pilze, Hefen, Algen und Schleimorganismen. Vorzugsweise wirken die erfindungsgemäß zu verwendenden Wirkstoffe gegen Pilze, insbesondere gegen Schimmelpilze sowie gegen Holz dauerhaft verfärbende und holzzerstörende Pilze;
also beispielsweise Mikroorganismen der folgenden Gattungen:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet können die erfindungsgemäß zu verwendenden Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Die zum Schutz technischer Materialien verwendeten mikrobiziden Mittel enthalten die Wirkstoffe im allgemeinen in einer Menge von 1 bis 100 Gew.-%, bevorzugt von 10 bis 90 Gew.-%.

Die Anwendungskonzentrationen der erfindungsgemäß zu verwendenden Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäß zu verwendenden Wirkstoffe können auch in Mischung mit anderen bekannten Wirkstoffen angewendet werden. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate, wie 2-Phenylphenol, (2,2'-Dihydroxy-5,5'-dichlor)-diphenylmethan und 3-Methyl-4-chlor-phenol, Organo-Zinnverbindungen, N-Trihalogenmethylthio-verbindungen, wie Folpet, Fluorfolpet und Dichlofluanid, Azolfungizide wie Triadimefon, Triadimenol, Bitertanol, Tebuconazole, Propiconazole, Iodpropargylverbindungen wie IPBC.

Auch Mischungen der erfindungsgemäß zu verwendenden Verbindungen I mit anderen bekannten Insektiziden können eingesetzt werden. Bevorzugte Beispiele für solche anderen Insektizide umfassen
Phosphorsäureester wie Azinphos-ethyl, Azinphos-methyl, 1-(4-Chlorphenyl)-4-(O-ethyl, S-propyl)phosphoryloxypyrazol (TIA-230), Chlorpyrifos, Coumaphos, Demeton, Demeton-S-methyl, Diazinon, Dichlorvos, Dimethoate, Ethoprophos, Etrimfos, Fenitrothion, Fenthion, Heptenophos, Parathion, Parathion-methyl, Phosalone, Pirimiphos-ethyl, Pirimiphos-methyl, Profenofos, Prothiofos, Sulprofos, Triazophos und Trichlorphon;
Carbamate wie Aldicarb, Bendiocarb, BPMC (2-(1-Methylpropyl)phenylmethylcarbamat), Butocarboxim, Butoxicarboxim, Carbaryl, Carbofuran, Carbosulfan, Cloethocarb, Isoprocarb, Methomyl, Oxamyl, Pirimicarb, Promecarb, Propoxur und Thiodicarb;
Pyrethroide wie Allethrin, Alphamethrin, Bioresmethrin, Byfenthrin (FMC 54 800), Cycloprothrin, Cyfluthrin, Cyhalothrin, Cypermethrin, Deltamethrin, Alpha-cyano-3-phenyl-2-methylbenzyl-2,2-dimethyl-3-(2-chlor-2-trifluormethylvinyl)cyclopropancarboxylat, Fenpropathrin, Fenfluthrin, Fenvalerate, Flucythrinate, Flumethrin, Fluvalinate, Permethrin und Resmethrin.

Die in den folgenden Beispielen vorkommenden Prozentangaben beziehen sich auf das Gewicht.

### Beispiele

### Beispiel 1-6 (3-monosubstituierte Verbindungen)

4,6 g (0,05 Mol) Thiosemicarbazid und 7,9 g (0,05 Mol) Butyrylessigsäureethylester werden zusammen mit 100 ml Ethanol 3 Stunden auf Rückflußtemperatur gehalten. Dann läßt man auf Raumtemperatur abkühlen und gibt unter Rühren portionsweise 5,9 g (0,05 Mol, 97 %ig) Kalium-tert.-butylat zu, um danach 4 Stunden bei Raumtemperatur weiterzurühren. Anschließend rührt man den Kolbeninhalt in ein Gemisch aus 800 ml Wasser/50 ml conc. HCl ein und saugt den ausgefallenen Niederschlag ab. Nach gründlichem Waschen mit Wasser wird das feuchte Produkt im Trockenschrank (50 mbar/60°C) bis zur Gewichtskonstanz belassen.
- Ausbeute:: 6,9 g (74,5 % d. Th.)
- Fp.:: 160-161°C
farbloser Feststoff.

In gleicher Weise können aus den entsprechenden Acylessigsäureethylestern die in der nachstehenden Tabelle 1 aufgeführten, in 3-Stellung substituierten 1-Thiocarbamoyl-5-hydroxy-pyrazole erhalten werden.

Die Charakterisierung der anfallenden Pyrazole erfolgt durch Schmelzpunktsbestimmung.

**Tabelle 1**

| Beispiele | R³ | R⁴ | R¹,R² | Fp. |
|---|---|---|---|---|
| 1 | C₃H₇ | H | H | 160-161°C |
| 2 | CH₃ | H | H | 178°C |
| 3 | C₂H₅ | H | H | 154-158°C |
| 4 | C₆H₁₃ | H | H | 142-143°C |
| 5 | C₈H₁₇ | H | H | 134-135°C |
| 6 | C₁₀H₂₁ | H | H | 154°C |

### Beispiele 7-14 (3,4-disubstituierte Verbindungen)

Ein Gemisch aus 3,9 g (0,025 Mol) 2-Ethyl-acetessigsäureethylester, 2,3 g (0,025 Mol) Thiosemicarbazid und 100 ml Ethanol wird unter Rühren 3 Stunden auf Rückflußtemperatur gehalten. Anschließend wird das Reaktionsgemisch auf 20°C abgekühlt und unter Rühren portionsweise mit 2,9 g (0,025 Mol; 97 %ig) Kalium-tert.-butylat versetzt. Die erhaltene Aufschlämmung wird dann 3 Stunden bei 20-30°C gerührt, anschließend in verdünnte Salzsäure (500 ml Wasser/20 ml conc. HCl) eingerührt und der Niederschlag abgesaugt. Man wäscht gründlich mit Wasser und trocknet in der Wärme bis zur Gewichtskonstanz.
- Ausbeute:: 2,9 g (82,5 % d. Th.)
- Fp.:: 163°C

In gleicher Weise können aus den entsprechenden α-substituierten β-Ketoestern die in der nachstehenden Tabelle 2 aufgeführten 3,4-disubstituierten 1-Thiocarbamoyl-5-hydroxypyrazole erhalten werden.

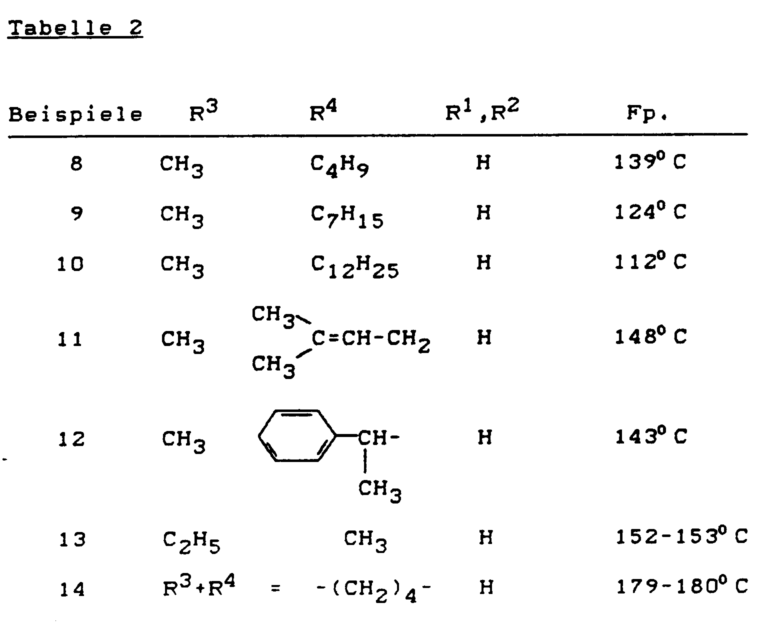

### Beispiele 15-42

10,3 g (0,06 Mol) α-Formyl-hexansäureethylester und 5,5 g (0,06 Mol) Thiosemicarbazid werden in 200 ml Ethanol vorgelegt und 3 Stunden bei 80°C gerührt.

Dann wird auf Raumtemperatur gebracht und unter Rühren 6,9 g Kalium-tert.-butylat zugegeben, um danach 4 Stunden bei Raumtemperatur weiterzurühren. Anschließend wird der Kolbeninhalt in ein Gemisch aus 800 ml Wasser und 50 ml conc. Salzsäure eingerührt und der erhaltene Niederschlag abgesaugt. Nach gründlichem Waschen mit Wasser wird das Produkt im Trockenschrank bis zur Gewichtskonstanz belassen. Durch Umkristallisieren aus Ethanol kann die Verbindung gereinigt werden.
- Ausbeute:: 9,4 g (80,3 % d. Th.)
- Fp.:: 139-141°C

In gleicher Weise können aus den entsprechenden α-Formyl-carbonsäureestern die in der nachfolgenden Tabelle 3 aufgeführten, 4-substituierten 1-Thiocarbamoyl-5-hydroxypyrazole erhalten werden.

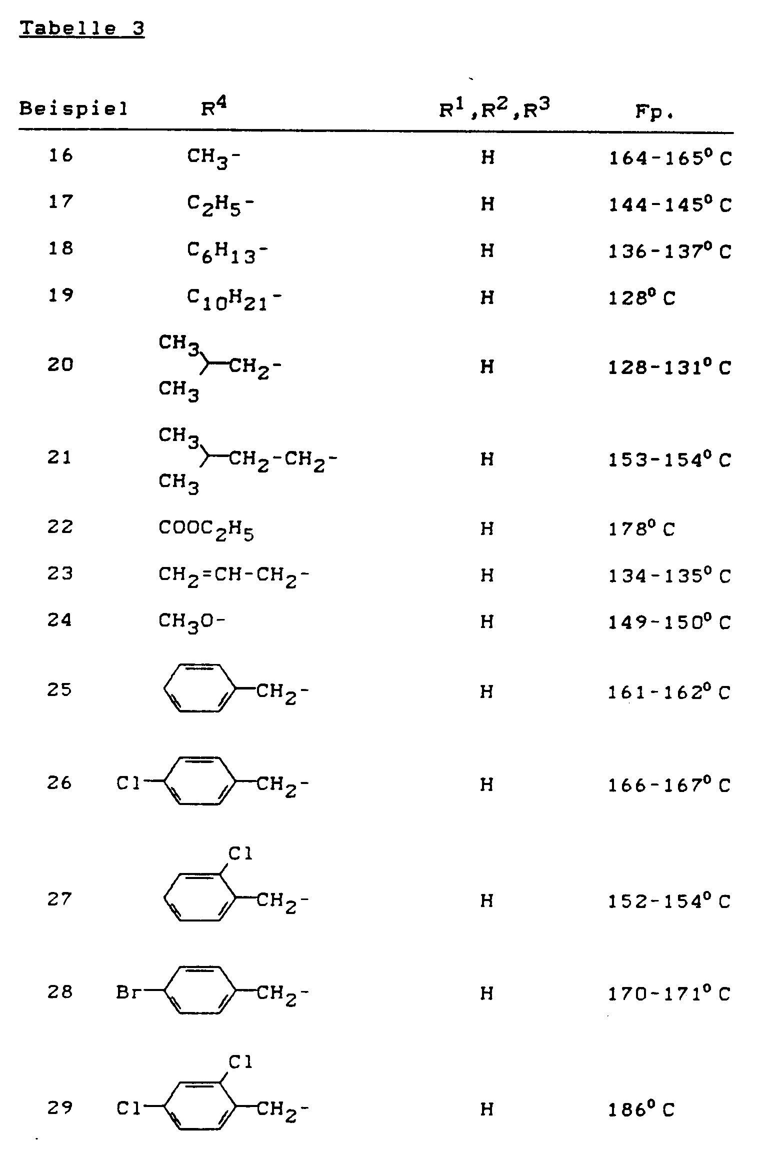

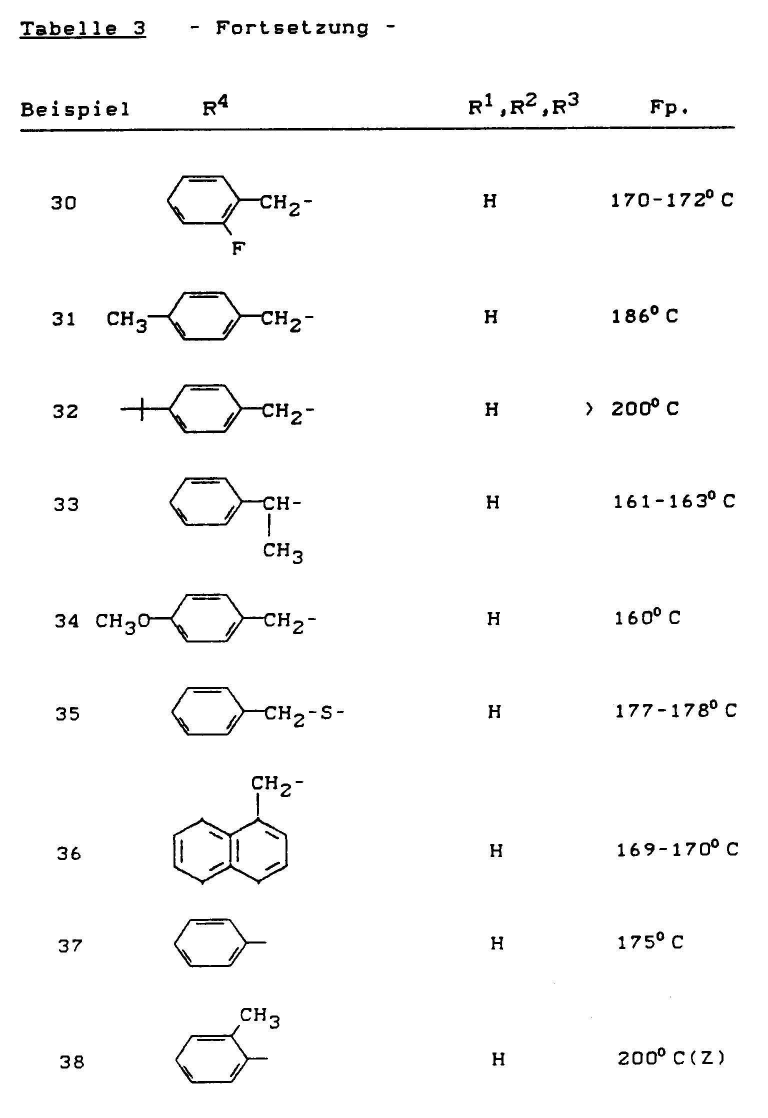

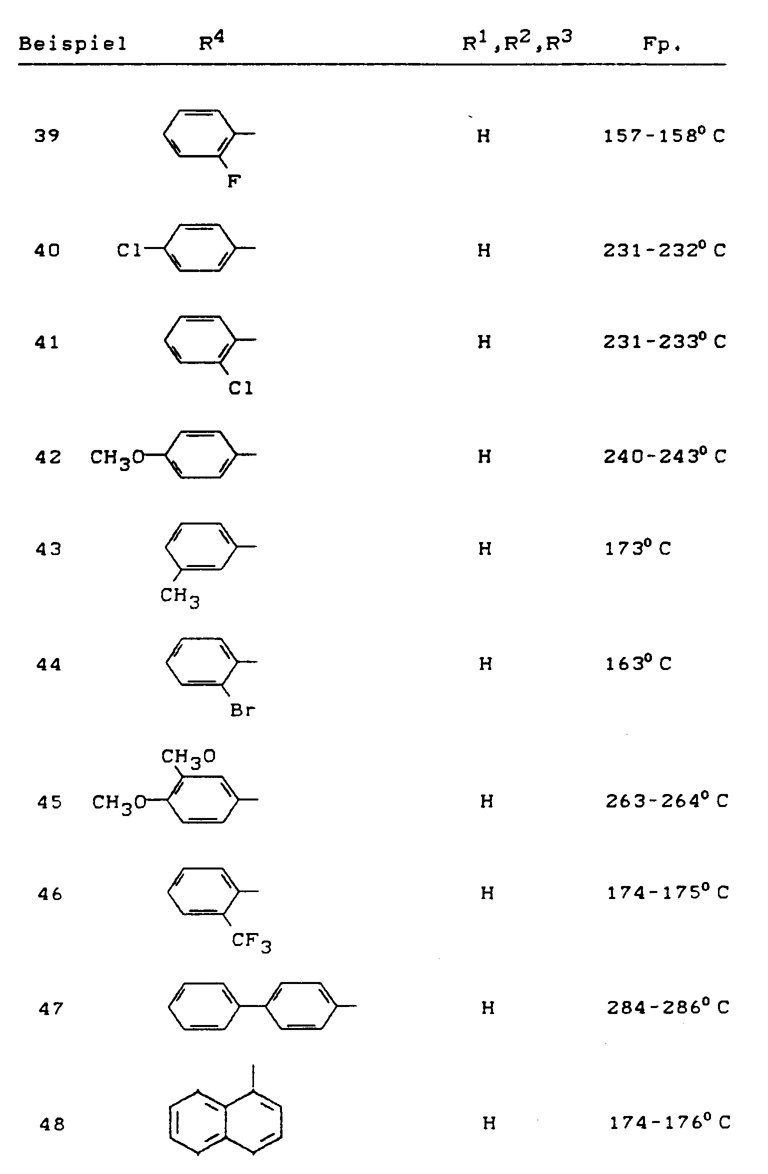

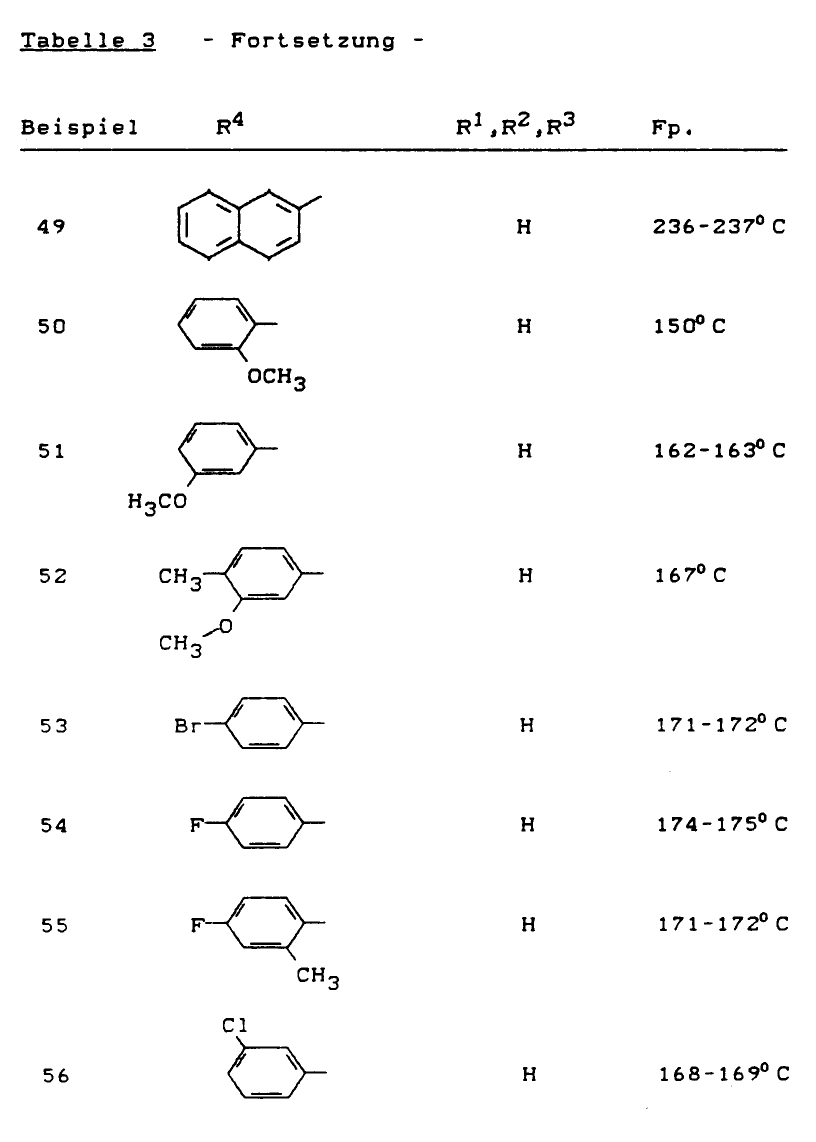

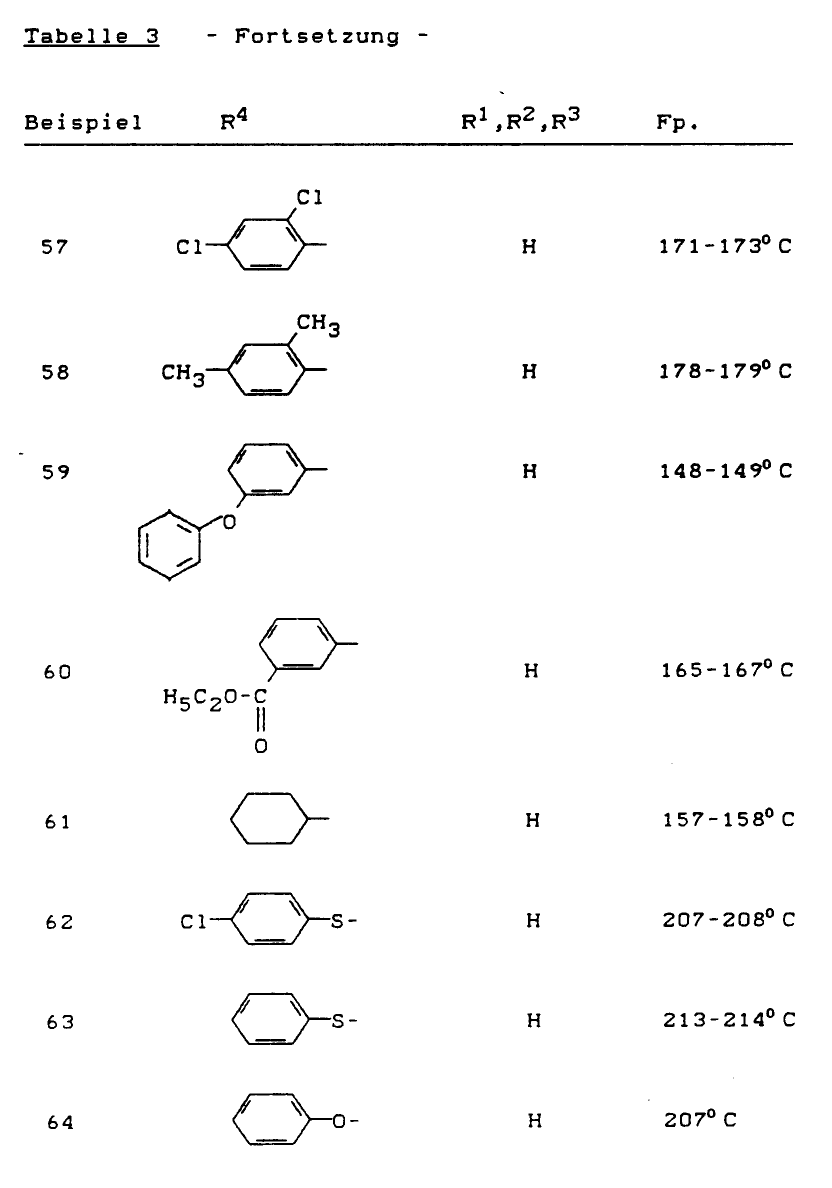

### Anwendungsbeispiele

### Beispiel A

Zum Nachweis der Wirksamkeit gegen Pilze wurden die minimalen Hemm-Konzentrationen (MHK) der erfindungsgemäß zu verwendenden Verbindungen bestimmt:

Ein Agar, der aus Bierwürze und Pepton hergestellt wird, wird mit erfindungsgemäß zu verwendenden Wirkstoffen in Konzentrationen von 0,1 mg/1 bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle 4 angegeben.

**Tabelle 4**

| MHK Werte [mg/l] verschiedener 1-Thiocarbamoyl-5-hydroxy-pyrazole auf Pilze | | | | | | | | |
|---|---|---|---|---|---|---|---|---|
| Testorganismen | 1-Thiocarbamoyl-5-hydroxy-pyrazole gemäß Beispiel | | | | | | | |
| | 15 | 21 | 23 | 25 | 26 | 27 | 33 | 34 |
| Alternaria tenuis | 5 | 10 | 50 | 50 | 50 | 50 | 50 | 50 |
| Aspergillus niger | 50 | 20 | 75 | 100 | 50 | 75 | 50 | 100 |
| Aureobasidium pullulans | 5 | 15 | 15 | 20 | 20 | 15 | 50 | 50 |
| Chaetomium globosum | 10 | <10 | 20 | 20 | 15 | 20 | 75 | 20 |
| Cladosporium herbarum | 20 | 20 | 75 | 50 | 50 | 50 | 50 | 50 |
| Lentinus tigrinus | 20 | <10 | 50 | 50 | <10 | 20 | 20 | 20 |
| Penicillium brevicaule | 20 | 15 | 20 | 50 | 15 | 75 | 75 | 75 |
| Sclerophoma pityophila | 15 | 15 | 75 | 50 | 100 | 35 | 35 | 50 |
| Trichoderma viride | 20 | 35 | 50 | 75 | 50 | 35 | 50 | 100 |

### Beispiel B (Fungizide Wirkung in Anstrichmitteln)

Die fungizide Wirkung in Anstrichmitteln wird durch Prüfung der Schimmelfestigkeit der mit den Anstrichmitteln erhaltenen Anstriche bestimmt.

Die Prüfung wird in Anlehnung an den Report 219 der Defense Standards Laboratories Maribyrnong/Australien wie folgt durchgeführt:

Das zu prüfende Anstrichmittel wird beidseitig auf eine geeignete Unterlage gestrichen.

Um praxisnahe Ergebnisse zu erhalten, wird ein Teil der Prüflinge vor dem Test auf Schimmelfestigkeit mit einem warmen Frischluftstrom behandelt (7 Tage; 40°C).

Die so vorbereiteten Prüflinge werden auf einen Agar-Nährboden gelegt. Prüfling und Nährboden werden mit Pilzsporen kontaminiert. Nach 1- bis 3-wöchiger Lagerung bei 29 ± 1°C und 80 bis 90 % rel. Luftfeuchte wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden:
1. Alternaria tenuis
2. Aspergillus flavus
3. Aspergillus niger
4. Aspergillus ustus
5. Cladosporium herbarum
6. Paecilomyces variotii
7. Penicillium citrinum
8. Aureobasidium pullulans
9. Stachybotrys atra Corda

In Proben einer handelsüblichen Dispersionsfarbe auf Polyvinylacetatbasis wurden 0 bis 2 Gew.-%, bezogen auf den Gesamtfeststoffgehalt der Farbe, der auf ihre fungizide Wirksamkeit zu prüfenden Verbindung eingearbeitet. Bei diesem Test lieferten bereits die Proben, die nur 0,3 Gew.-%, bezogen auf Gesamtfeststoffgehalt der Farbe, des in Beispiel 15 beschriebenen 1-Thiocarbamoyl-4-n-butyl-5-hydroxy-pyrazols enthielten, farblose dauerhaft schimmelfeste Anstriche.

## Patentansprüche

1. Verwendung von Verbindungen der Formel 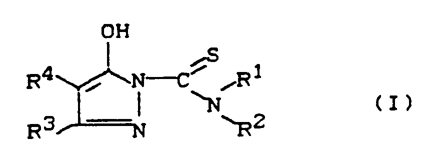 worin
R¹ und R² unabhängig voneinander Wasserstoff oder gegebenenfalls substituiertes Alkyl,
R³ Wasserstoff oder gegebenenfalls substituiertes Alkyl, Alkenyl, Alkinyl, Cycloalkyl, Alkoxycarbonyl, Aralkyl oder Aryl,
R⁴ Wasserstoff oder gegebenenfalls substituiertes Alkyl, Alkenyl, Alkinyl, Cycloalkyl, Alkoxy, Alkylthio, Aralkyl, Aralkoxy, Aralkylthio, Aryl, Aryloxy, Arylthio, Alkoxycarbonyl, Alkoxycarbonylmethyl oder Aminoycarbonyl oder
R³ und R⁴ zusammen einen 1,ω-C₃-C₆-Alk(en)ylenrest bedeuten,
als Mikrobizide zum Schutz technischer Materialien.

2. Verwendung gemäß Anspruch 1 von solchen Verbindungen der Formel I, in der
R¹ Wasserstoff oder gegebenenfalls substituiertes Alkyl, Aralkyl oder Aryl,
R² Wasserstoff,
R³ Wasserstoff oder gegebenenfalls substituiertes Alkyl oder Aryl und
R⁴ Wasserstoff oder gegebenenfalls substituiertes Alkyl, Cycloalkyl, Aralkyl, Alkoxycarbonyl oder Aryl bedeuten.

3. Verwendung gemäß Anspruch 1 von solchen Verbindungen der Formel I, in der
R¹-R³ Wasserstoff und
R⁴ Wasserstoff oder gegebenenfalls substituiertes Alkyl, Aralkyl oder Aryl bedeuten.

4. Verwendung gemäß Anspruch 1 von solchen Verbindungen der Formel I, in der
R¹-R³ Wasserstoff und
R⁴ C₁-C₆-Alkyl, Benzyl oder Phenyl bedeuten.

5. Verwendung nach einem der Ansprüche 1 bis 4, dadurch gekennzeichnet, daß die technischen Materialien Anstrichmittel, Kunststoffe oder Kunststoffartikel sind.

6. Verbindungen der Formel 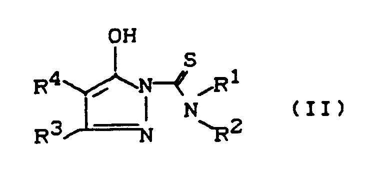 worin
R¹ und R² die im Anspruch 1 angegebene Bedeutung besitzen,
R³ für Wasserstoff und
R⁴ für gegebenenfalls substituiertes C₆-C₈-Alkyl, gegebenenfalls substituiertes Aryl mit Ausnahme von Phenyl und 4-Chlorphenyl, oder
für gegebenenfalls substituiertes Aralkyl mit Ausnahme von unsubstituiertem Benzyl stehen.

7. Verfahren zur Herstellung der Verbindungen gemäß Anspruch 6,
wonach man Verbindungen der Formeln 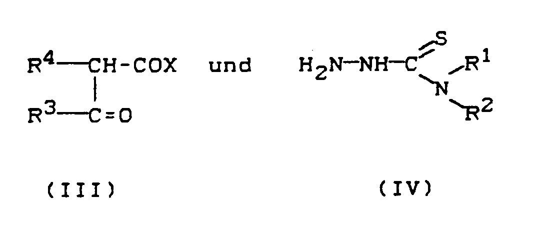 worin
R¹ bis R⁴ die in Anspruch 6 angegebene Bedeutung besitzen und
X für C₁-C₄-Alkoxy, Phenoxy oder Amino steht,
auf an sich bekannte Weise umsetzt.

## Claims

1. Use of compounds of the formula 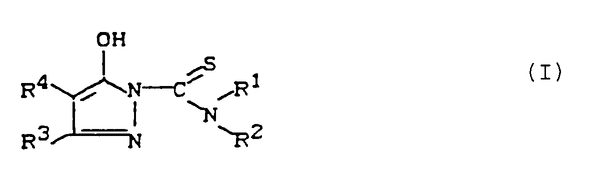 wherein
R¹ and R² independently of one another denote hydrogen or optionally substituted alkyl,
R³ denotes hydrogen or optionally substituted alkyl, alkenyl, alkinyl, cycloalkyl, alkoxycarbonyl, aralkyl or aryl,
R⁴ denotes hydrogen or optionally substituted alkyl, alkenyl, alkinyl, cycloalkyl, alkoxy, alkylthio, aralkyl, aralkoxy, aralkylthio, aryl, aryloxy, arylthio, alkoxycarbonyl, alkoxycarbonylmethyl or aminocarbonyl, or
R³ and R⁴ together denote a 1,ω-C₃-C₆-alk(en)ylene radical,
as microbicides for the preservation of industrial materials.

2. Use according to Claim 1 of those compounds of the formula I, in which
R¹ denotes hydrogen or optionally substituted alkyl, aralkyl or aryl,
R² denotes hydrogen,
R³ denotes hydrogen or optionally substituted alkyl or aryl and
R⁴ denotes hydrogen or optionally substituted alkyl, cycloalkyl, aralkyl, alkoxycarbonyl or aryl.

3. Use according to Claim 1 of those compounds of the formula I, in which
R¹-R³ denote hydrogen and
R⁴ denotes hydrogen or optionally substituted alkyl, aralkyl or aryl.

4. Use according to Claim 1 of those compounds of the formula I, in which
R¹-R³ denote hydrogen and
R⁴ denotes C₁-C₆-alkyl, benzyl or phenyl.

5. Use according to one of Claims 1 to 4, characterised in that the industrial materials are paints, plastics or articles made of plastic.

6. Compounds of the formula 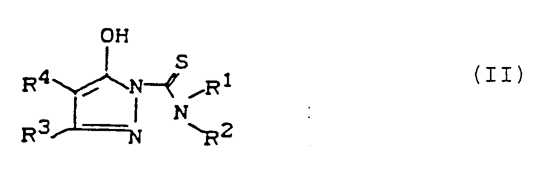 wherein
R¹ and R² have the meaning given in Claim 1,
R³ represents hydrogen and
R⁴ represents optionally substituted C₆-C₈-alkyl, optionally substituted aryl, with the exception of phenyl and 4-chlorophenyl, or
optionally substituted aralkyl, with the exception of unsubstituted benzyl.

7. Process for the preparation of the compounds according to Claim 6,
in which compounds of the formulae 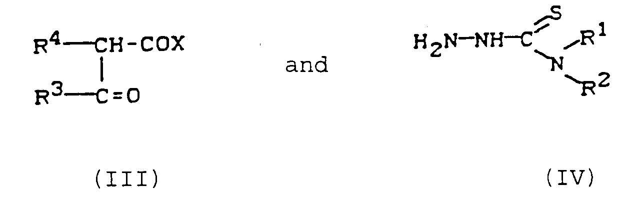 wherein
R¹ to R⁴ have the meaning given in Claim 6 and
X represents C₁-C₄-alkoxy, phenoxy or amino, are reacted in a manner which is known per se.

## Revendications

1. Utilisation des composés de formule 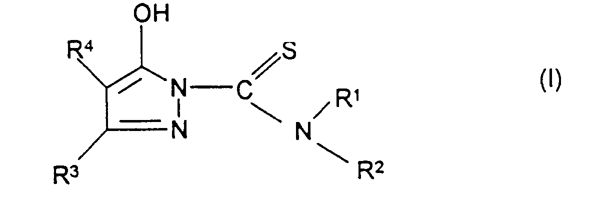 dans laquelle
R¹ et R² représentent chacun, indépendamment l'un de l'autre, l'hydrogène ou un groupe alkyle éventuellement substitué,
R³ représente l'hydrogène ou un groupe alkyle, alcényle, alcynyle, cycloalkyle, alcoxycarbonyle, aralkyle ou aryle éventuellement substitué,
R⁴ représente l'hydrogène ou un groupe alkyle, alcényle, alcynyle, cycloalkyle, alcoxy, alkylthio, aralkyle, aralcoxy, aralkylthio, aryle, aryloxy, arylthio, alcoxycarbonyle, alcoxycarbonylméthyle ou aminoycarbonyle éventuellement substitué,
ou bien
R³ et R⁴ représentent ensemble un groupe 1,ω-alkylène en C₃-C₆, ou 1,ω-alcénylène en C₃-C₆,
en tant que microbicides pour la protection des matériaux techniques.

2. Utilisation selon la revendication 1 de composés de formule I dans laquelle
R¹ représente l'hydrogène ou un groupe alkyle, aralkyle ou aryle éventuellement substitué,
R² représente l'hydrogène,
R³ représente l'hydrogène ou un groupe alkyle ou aryle éventuellement substitué et
R⁴ représente l'hydrogène ou un groupe alkyle, cycloalkyle, aralkyle, alcoxycarbonyle ou aryle éventuellement substitué.

3. Utilisation selon la revendication 1 de composés de formule (I) dans laquelle
R¹ à R³ représentent l'hydrogène et
R⁴ représente l'hydrogène ou un groupe alkyle, aralkyle ou aryle éventuellement substitué.

4. Utilisation selon la revendication 1, de composés de formule (I) dans laquelle
R¹ à R³ représentent l'hydrogène et
R⁴ représente un groupe alkyle en C₁-C₆, benzyle ou phényle.

5. Utilisation selon l'une des revendications 1 à 4, caractérisée en ce que les matériaux techniques sont des produits de revêtement, des résines synthétiques ou des articles en résines synthétiques.

6. Composés de formule 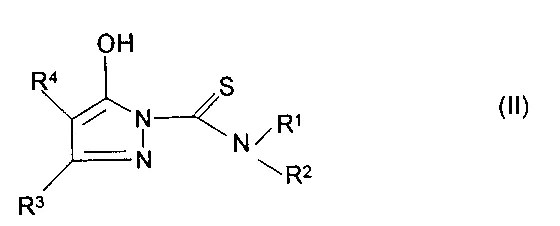 dans laquelle
R¹ et R² ont les significations indiquées dans la revendication 1,
R³ représente l'hydrogène et
R⁴ représente un groupe alkyle éventuellement substitué en C₆-C₈, un groupe aryle éventuellement substitué en C₆-C₁₀, à l'exclusion des groupes phényle et 4-chlorophényle, ou bien
un groupe aralkyle éventuellement substitué, à l'exclusion du groupe benzyle non substitué

7. Procédé de préparation des composés de la revendication 6,
selon lequel on fait réagir de manière connue en soi des composés de formules 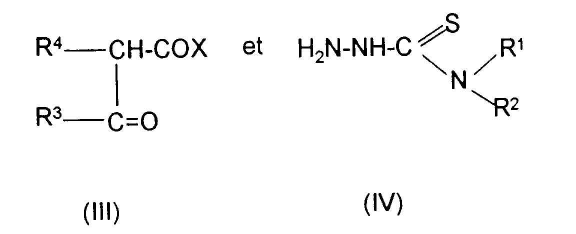 dans lesquelles les symboles
R¹ à R⁴ ont les significations indiquées dans la revendication 6 et
X représente un groupe alcoxy en C₁-C₄, phénoxy ou amino.
